# EUROPÄISCHE PATENTSCHRIFT

(11) **EP 1 786 403 B1**
(45) Veröffentlichungstag und Bekanntmachung des Hinweises auf die Patenterteilung: **24.07.2013**
(21) Anmeldenummer: 05758495.5
(22) Anmeldetag: 29.06.2005
(51) Int. Cl.: A61K 9/22, A61K 31/135

(54) **GEGEN MISSBRAUCH GESICHERTE, ORALE DARREICHUNGSFORM ENTHALTEND (1R, 2R)-3-(3-DIMETHYLAMINO-1-ETHYL-2-METHYL-PROPYL)-PHENOL**
ORAL DOSAGE FORM SAFEGUARDED AGAINST ABUSE CONTAINING (1R, 2R)-3-(3-DIMETHYLAMINO-1-ETHYL-2-METHYL-PROPYL)-PHENOL
FORME POSOLOGIQUE ANTI-ABUS POUR ADMINISTRATION PAR VOIE ORALE CONTENANT DU (1R, 2R)-3-(3-DIMETHYLAMINO-1-ETHYL-2-METHYL-PROPYL)-PHENOL

(30) Priorität: 01.07.2004 DE 102004032103; 14.07.2004 US 890707
(43) Veröffentlichungstag der Anmeldung: 23.05.2007
(73) Patentinhaber: Grünenthal GmbH, 52078 Aachen (DE)
(72) Erfinder: BARTHOLOMÄUS, Johannes Dr., 52080 Aachen (DE); KUGELMANN, Heinrich, 52068 Aachen (DE); ARKENAU-MARIC, Elisabeth Dr., 50931 Köln (DE)
(74) Vertreter: Kutzenberger, Helga
(86) Internationale Anmeldenummer: PCT/EP2005/006990
(87) Internationale Veröffentlichungsnummer: WO 2006/002886

(56) Entgegenhaltungen:
- WO-A-97/33566
- WO-A-03/035053
- WO-A-2004/037230
- WO-A-2004/037259
- WO-A-2004/037260
- DE-A1- 10 250 083
- US-A1- 2003 068 392
- US-A1- 2003 124 185

## Beschreibung

Die vorliegende Erfindung betrifft eine gegen Missbrauch gesicherte, orale Darreichungsform mit kontrollierter Freisetzung des Wirkstoffs (1 R, 2R)-3-(3-dimethylamino-1-ethyl-2-methyl-propyl)-phenol für eine einmal tägliche Verabreichung, die den Wirkstoff und/oder eines oder mehrere seiner pharmazeutisch akzeptablen Salzen und/oder Derivate (A), mindestens ein synthetisches oder natürliches Polymer (C), ggf. retardierende Matrix-Materialien, ggf. physiologisch verträgliche Hilfsstoffe (B) und gegebenenfalls ein Wachs (D) umfasst, wobei die Darreichungsform eine Bruchfestigkeit von mindestens 500 N aufweist.

Dieser Wirkstoff weist neben seiner ausgezeichneten schmerzlindernden Wirkung auch ein Missbrauchspotential auf, d. h. es kann von einem Missbraucher eingesetzt werden, um Wirkungen herbeizuführen, die nicht seinem Bestimmungszweck entsprechen. So wird dieser Wirkstoff von Missbrauchem beispielsweise zum Einleiten rauschartiger, euphorisierender Zustände verwendet.

Diese wirkstoffhaltigen Darreichungsformen werden häufig in Langzeittherapien eingesetzt, beispielsweise bei tumorbedingten oder chronisch bedingten Schmerzen. Insbesondere bei einer Langzeittherapie ist es wichtig, dem Patienten eine gute Lebensqualität zu ermöglichen. Zu den Maßnahmen, die die Lebensqualität eines Patienten erhöhen, gehören u. a. Darreichungsformen, die eine einmal tägliche Verabreichung erlauben. Solche Darreichungsformen, die den Wirkstoff retardiert freisetzen, sind aber wegen der relativ hohen Menge des Wirkstoffs für den Missbraucher besonders attraktiv, um die gewünschten, rauschartigen, euphorisierenden Zustände möglichst rasch herbei führen zu können.

Da aber retardierte Darreichungsformen, die den genannten Wirkstoff enthalten, üblicherweise selbst bei einer oralen Einnahme von missbräuchlich hohen Mengen nicht zu dem vom Missbraucher gewünschten Kick führen, werden diese beispielsweise in Form von Tabletten oder Kapseln vorliegenden Darreichungsformen für den Missbrauch vom Missbraucher zerkleinert, z. B. gemörsert, und geschnupft oder die Wirkstoffe vorzugsweise aus dem so erhaltenen Pulver mit Hilfe einer wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber dem oralen, aber auch gegenüber dem nasalen Missbrauch zusätzlich beschleunigten Anfluten des Wirkstoffs mit dem vom Missbraucher gewünschten Ergebnis, nämlich den Kick.

Zur Verhinderung von Missbrauch wurde in dem US-A- 4,070,494 vorgeschlagen, der Darreichungsform ein quellbares Mittel zuzusetzen. Dieses quillt bei der Zugabe von Wasser zur Extraktion des dort verwendeten Wirkstoffes auf und bewirkt, dass das vom Gel separierte Filtrat nur eine geringe Menge an Wirkstoff enthält.

Ein entsprechender Ansatz zur Verhinderung eines parenteralen Missbrauchs liegt auch der in der WO 95/20947 offenbarten Mehrschichttablette zugrunde, die einen Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll der Missbraucher davon abhalten, diese manipulierte Darreichungsform zu verwenden. WO 2004/037259 beschreibt den Zusatz von Reizstoffen zu Missbrauchsverbindung.

Eine weitere bekannte Möglichkeit zur Erschwerung des Missbrauchs besteht darin, der Darreichungsform einen Antagonisten des Wirkstoffes, wie z. B. Naloxon oder Naltrexon, oder Verbindungen, die zu physiologischen Abwehrreaktionen führen, wie z. B. Radix Ipecacuama = Brechwurz oder Bitterstoffe, der Darreichungsform zuzusetzen.

Da aber nach wie vor in den meisten Fällen für den Missbrauch von Darreichungsformen mit kontrollierter Freisetzung des Wirkstoffs eine Pulverisierung notwendig ist, war es Aufgabe der vorliegenden Erfindung, die dem Missbrauch vorangehende Pulverisierung der Darreichungsform mit den einem potentiellen Missbraucher üblicherweise zur Verfügung stehenden Mitteln zu erschweren bzw. zu verhindern, mit kontrollierter Freisetzung von (1R, 2R)-3-(3-dimethylamino-1-ethyl-2-methyl-propyl)-phenol und somit eine Darreichungsform für den Wirkstoff zur Verfügung zu stellen, die bei bestimmungsgemäßer Applikation die gewünschte therapeutische Wirkung bei einer einmal täglichen Verabreichung gewährleistet, aus der aber der Wirkstoff nicht durch einfaches Pulverisieren in eine zum Missbrauch geeignete Form übergeführt werden kann.

Diese Aufgabe wurde durch die Bereitstellung der erfindungsgemäßen, gegen Missbrauch gesicherten, oralen Darreichungsform mit kontrollierter Freisetzung von (1 R, 2R)-3-(3-dimethylamino-1-ethyl-2-methyl-propyl)-phenol für eine einmal tägliche Verabreichung, die neben dem Wirkstoff und/oder einem oder mehreren seiner pharmazeutisch akzeptablen Verbindungen, vorzugsweise Salze, Solvate oder Derivate, vorzugsweise Ester, Ether oder Amide sowie den entsprechenden Stereoisomeren und/oder deren entsprechende pharmazeutisch akzeptablen Verbindungen oder Derivaten (A), mindestens ein synthetisches und/oder natürliches Polymer (C), wenigstens einen retardierenden Hilfsstoff (E), ggf. wenigstens einem weiteren physiologisch verträglichen Hilfsstoff (B) und gegebenenfalls ein Wachs (D) umfasst, wobei die Komponente (C) bzw. (D) jeweils eine Bruchfestigkeit von mindestens 500 N, vorzugsweise mindestens 750 N, aufweist, gelöst.

Durch den Einsatz von Komponenten (C) und ggf. (D) mit der angegebenen Mindestbruchfestigkeit (gemessen wie in den vorliegenden Anmeldung offenbart), vorzugsweise in solchen Mengen, dass auch die Darreichungsform eine solche Mindestbruchfestigkeit von mindestens 500 N, vorzugsweise mindestens 750 N aufweist, gelingt es, ein Pulverisieren der Darreichungsform mit üblichen Mitteln zu verhindern und damit den anschließenden Missbrauch, vorzugsweise einen nasalen oder parenteralen Missbrauch, erheblich zu erschweren bzw. zu verhindern.

Ohne ausreichende Zerkleinerung der Darreichungsform ist nämlich eine gefahrlose, parenterale, insbesondere intravenöse oder eine nasale Applikation nicht möglich oder die Extraktion des Wirkstoffs daraus dauert dem Missbraucher zu lange bzw. ein Kick erfolgt bei missbräuchlicher, oraler Einnahme nicht, oder nicht in ausreichender Weise, da keine spontane Freisetzung passiert.

Unter einer Zerkleinerung wird erfindungsgemäß die Pulverisierung der Darreichungsform mit üblichen Mitteln, die einem Missbraucher üblicherweise zur Verfügung stehen wie z. B. ein Mörser und Pistill, ein Hammer, ein Schlegel oder andere gebräuchliche Mittel zum Pulverisieren unter Krafteinwirkung, verstanden.

Die erfindungsgemäße Darreichungsform ist daher zur Verhinderung des parenteralen, nasalen und/oder oralen Missbrauchs von (1 R, 2R)-3-(3-dimethylamino-1-ethyl-2-methyl-propyl)-phenol geeignet. Der Wirkstoff ist als ein analgetisch wirksames Arzneimittel aus EP-A-0 693 475 bekannt.

Dabei kann der Wirkstoff (1 R, 2R)-3-(3-dimethylamino-1-ethyl-2-methyl-propyl)-phenol als solches, d. h. als freie Base, aber auch in Form eines seiner pharmazeutisch akzeptablen Salze, Solvate, eines pharmazeutisch akzeptablen Derivates, insbesondere Amids, Esters oder Ethers, und/oder der entsprechenden Stereoisomeren und/oder deren entsprechende pharmazeutisch akzeptable Verbindung eingesetzt werden. Die Herstellung des Wirkstoffes ist auch aus EP-A- 0 693 475 A1 bekannt.

In der erfindungsgemäßen Darreichungsform liegt der Gehalt des Wirkstoffs vorzugsweise zwischen 0,5 und 80 Gew.-%, besonders bevorzugt zwischen 10 und 40 Gew.-% und ganz besonders bevorzugt zwischen 5-50 Gew.-%.

Die erfindungsgemäße Darreichungsform enthält (1 R, 2R)-3-(3-dimethylamino-1-ethyl-2-methyl-propyl)-phenol als solches und/oder als pharmazeutisch akzeptable Verbindung üblicherweise in einer Menge von 2,5 bis 1.000 mg, insbesondere 5 bis 800 mg, ganz besonders bevorzugt von 5-600 mg berechnet als (1 R, 2R)-3-(3-dimethylamino-1-ethyl-2-methyl-propyl)-phenol pro Darreichungsform bzw. Dosierungseinheit

Pharmazeutisch akzeptable Salze des Wirkstoffs sind erfindungsgemäß Salze, die bei pharmazeutischer Verwendung, insbesondere bei der bestimmungsgemäßen Verabreichung an Säugetiere oder Menschen, insbesondere an Menschen physiologisch verträglich sind. Als solche pharmazeutischen Salze können beispielsweise Salze mit anorganischen oder organischen Säuren wie z. B. vorzugsweise das Hydrochlorid, Hydrobromid, Saccharinat, Sulfat, das Salz mit der Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, ganz besonders bevorzugt wird als Salz das Hydrochlorid eingesetzt werden.

Zur Erzielung der notwendigen Bruchfestigkeit der erfindungsgemäßen Darreichungsform werden mindestens ein synthetisches, halbsynthetisches und/oder natürliches Polymer (C) mit einer Bruchfestigkeit, gemessen nach der in der vorliegenden Anmeldung offenbarten Methode, von mindestens 500 N, vorzugsweise 750 N, eingesetzt. Bevorzugt wird hierfür mindestens ein Polymeres ausgewählt aus der Gruppe umfassend Polyalkylenoxide, vorzugsweise Polymethylenoxide, Polyethylenoxide, Polypropylenoxide, Polyolefine, vorzugsweise Polyethylene, Polypropylene, Polyvinylchloride, Polycarbonate, Polystyrole, Poly(meth)acrylate, deren Copolymerisate und Mischungen aus mindestens zwei Vertretern der genannten Polymerklassen oder Polymeren eingesetzt. Besonders bevorzugt wird ein wasserlösliches oder wasserquellbares Polymer eingesetzt. Bevorzugt sind hochmolekulare, thermoplastische Polyalkylenoxide. Besonders bevorzugt sind Polyethylenoxide mit einem Molekulargewicht von mindestens 0,5 Mio., vorzugsweise von mindestens 1 Mio., besonders bevorzugt von 1 Mio. bis 15 Mio., bestimmt durch rheologische Messungen. Diese Polyethylenoxide weisen eine Viskosität bei 25 °C von 4500 bis 17600 cP, gemessen an einer 5 Gew.% wässrigen Lösung des Polymeren mit Hilfe eines Brookfield Viskosimeters, Model RVF (Spindel Nr. 2 / Rotationsgeschwindigkeit 2 rpm), von 400 bis 4000 cP, gemessen an einer 2 Gew.% wässrigen Lösung des Polymeren mit Hilfe des genannten Viskosimeters (aber mit Spindel Nr. 1 bzw. 3 / Rotationsgeschwindigkeit 10 rpm) bzw. von 1650 bis 10000 cP, gemessen an einer 1 Gew.% wässrigen Lösung des Polymers mit Hilfe des genannten Viskosimeters (aber mit Spindel Nr. 2 /Rotationsgeschwindigkeit 2 rpm) auf (vgl. Handbook of Pharmaceutical Excipients von Raymond C. Rowe u. a., Ausgabe 4., 2003, Seite 460).

Die Polymeren werden vorzugsweise als Pulver zur Herstellung der erfindungsgemäßen Darreichungsform eingesetzt. Sie können wasserlöslich oder wasserquellbar sein.

Vorzugsweise wird die Komponente (C) in einer Menge von 20 bis 99,9 Gew.%, besonders bevorzugt von wenigstens 35 Gew.%, ganz besonders bevorzugt von wenigstens 50 Gew.%, bezogen auf das Gesamtgewicht der Darreichungsform, eingesetzt.

Als Hilfsstoffe (B) können die üblichen für die Formulierung von festen Darreichungsformen bekannte Hilfsstoffe verwendet werden. Vorzugsweise sind diese Weichmacher, wie Polyethylenglykol, Hilfsstoffe, die die Wirkstofffreisetzung beeinflussen, wie nachstehend aufgeführt, vorzugsweise hydrophobe oder hydrophile, vorzugsweise hydrophile Polymere, ganz besonders bevorzugt Hydroxypropylmethylcellulose oder Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Des weiteren können neben den vorstehend genannten Polymeren zusätzlich zur Erzielung der notwendigen Bruchfestigkeit der erfindungsgemäßen Darreichungsform mindestens ein natürliches, halbsynthetisches oder synthetisches Wachs (D) mit einer Bruchfestigkeit, gemessen nach der in der vorliegenden Anmeldung offenbarten Methode von mindestens 500 N, vorzugsweise 750 N, eingesetzt werden. Bevorzugt sind Wachse mit einem Erweichungspunkt von mindestens 60°C. Besonders bevorzugt sind Carnaubawachs und Bienenwachs. Ganz besonders bevorzugt ist Carnaubawachs. Carnaubawachs ist ein natürliches Wachs, das aus den Blättern der Camaubapalme gewonnen wird und einen Erweichungspunkt von höchstens 90°C aufweist. Bei einem zusätzlichen Einsatz der Wachskomponente wird diese zusammen mit wenigstens einem Polymeren (C), vorzugsweise mit wenigstens einem Polyethylenoxid, in solchen Mengen eingesetzt, dass die Darreichungsform eine Bruchfestigkeit von mindestens 500 N, vorzugsweise mindestens 750 N, gemessen nach der in der vorliegenden Anmeldung angegebenen Methode, aufweist.

Die erfindungsgemäßen Darreichungsformen zeichnen sich dadurch aus, dass sie aufgrund ihrer Härte nicht mit Hilfe von üblichen Zerkleinerungsmitteln, wie Mörser und Pistill zu pulverisieren sind. Ein oraler, parenteraler, insbesondere intravenöser oder nasaler Missbrauch ist dadurch praktisch ausgeschlossen. Um jedoch jeden möglichen Missbrauch der erfindungsgemäßen Darreichungsformen vorzubeugen, können die erfindungsgemäßen Darreichungsformen in einer bevorzugten Ausführungsform als Hilfsstoffe (B) weitere missbrauchs-erschwerende bzw. - verhindernde Mittel enthalten.

So kann die erfindungsgemäße, gegen Missbrauch gesicherte Darreichungsform, die neben dem erfindungsgemäß zum Einsatz kommenden Wirkstoff mindestens einem Polymer (C) und ggf. mindestens einem Wachs (D) noch wenigstens eine der nachfolgenden Komponenten (a)-(e) als Hilfsstoffe (B) aufweisen:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit, vorzugsweise als ein aus der Darreichungsform gewonnenes wässriges Extrakt, ein Gel bildet, welches vorzugsweise beim Einbringen in eine weitere Menge einer wässrigen Flüssigkeit visuell unterscheidbar bleibt,
(c) wenigstens einen Antagonisten für den zum Einsatz kommenden Wirkstoff,
(d) wenigstens ein Emetikum,
(e) wenigstens einen Farbstoff als aversives Mittel,
(f) wenigstens einen Bitterstoff.

Die Komponenten (a) bis (f) sind jede für sich allein zusätzlich als zusätzliche Sicherung der erfindungsgemäßen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die Mitverwendung von wenigstens einer der vorstehend genannten Komponenten, gelingt es, bei den erfindungsgemäßen Darreichungsformen noch effektiver den Missbrauch zu erschweren.

In einer Ausführungsform kann die erfindungsgemäße Darreichungsform auch zwei oder mehrere der Komponenten (a)-(f) in einer Kombination aufweisen, vorzugsweise in den Kombinationen (a), (b) und ggf. (c) und/oder (f) und/oder (e) bzw. (a), (b) und ggf. (d) und/oder (f) und/oder (e).

In einer weiteren Ausführungsform kann die erfindungsgemäße Darreichungsform sämtliche Komponenten (a)-(f) aufweisen.

Sofern die erfindungsgemäße Darreichungsform als zusätzlichen Schutz gegen Missbrauch die Komponente (a) umfaßt, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass der die Applikation nicht weiter fortsetzen will oder kann, z. B. ein Brennen, oder die auf physiologische Art und Weise einer Aufnahme des Wirkstoffs entgegenwirken, z. B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so dass der Missbraucher die Applikation nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Unter Darreichungseinheit wird eine separate bzw. separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäße Darreichungsform auch Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.

Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.-%, bezogen auf das Gesamtgewicht der Darreichungseinheit.

Eine weitere Möglichkeit, bei der erfindungsgemäßen Darreichungsform zusätzlich gegen Missbrauch vorzubeugen, besteht darin, wenigstens ein viskositätserhöhendes Mittel als weitere Missbrauchs-verhindemde Komponente (b) der Darreichungsform zuzusetzen, die in einer notwendigen Mindestmenge an einer wässrigen Flüssigkeit, vorzugsweise als ein aus der Darreichungsform gewonnenes wässriges Extrakt, ein Gel bildet, das kaum gefahrlos applizierbar ist und vorzugsweise auch beim Einbringen in eine weitere Menge einer wässrigen Flüssigkeit visuell unterscheidbar bleibt.

Visuelle Unterscheidbarkeit im Sinne der vorliegenden Anmeldung bedeutet, dass das mit Hilfe einer notwendigen Mindestmenge an wässriger Flüssigkeit gebildete, wirkstoffhaltige Gel beim Einbringen, vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung zum Gel führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wäßriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. In Kombination mit mindestens einer weiteren vorhandenen Komponente (a), (d) bis (f) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schaden des Missbrauchers führen.

Zur Überprüfung, ob ein viskositätserhöhendes Mittel als Komponente (b) zur Anwendung in der erfindungsgemäßen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur zusätzlichen Missbrauchs-Vorbeugung bzw. - Verhinderung bei den erfindungsgemäßen Darreichungsformen.

Sofern die durch das erfindungsgemäße Verfahren erhaltene Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLG-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401)^{®}) Guarkemmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakemmehl (Polygum 43/1^{®}), Propylenglykoalginat (Protanal-Ester SD-LB^{®}), Natrium-Hyaluronat, Tragant, Taragummi (Vidogum SP 200^{®}), fermentiertes Polysaccharid- Welan Gum (K1A96), Xanthane wie Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist vorzugsweise eine Menge von 0,1 bis 20 Gew.%, besonders bevorzugt 0,1 bis 15 Gew.%, bezogen auf die Gesamtmenge der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von mindestens 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die vorzugsweise durch Extraktion aus der Darreichungsform mit der notwendigen Mindestmenge an wässriger Flüssigkeit ein Gel bilden, das Luftblasen einschließt Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge an einer wässrigen Flüssigkeit ein Gel bildet.

Es ist auch möglich, die viskositätserhöhende Komponente und die übrigen Bestandteile der erfindungsgemäßen Darreichungsform voneinander räumlich getrennt anzuordnen.

Des weiteren kann die erfindungsgemäße Darreichungsform zusätzlich zur Vorbeugung und Sicherung gegen Missbrauch die Komponente (c) aufweisen, nämlich einen oder mehrere Antagonisten des zum Einsatz kommenden Wirkstoffs, wobei der Antagonist vorzugsweise räumlich getrennt von den übrigen Bestandteilen der erfindungsgemäßen Darreichungsform angeordnet ist und keine Wirkung bei bestimmungsgemäßer Verwendung entfalten soll.

Geeignete Antagonisten zur Verhinderung des Missbrauchs des verwendeten Wirkstoffs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Als Antagonist kommt bevorzugt ein Stoff ausgewählt aus der Gruppe umfassend Naloxon, Naltrexon, Nalmefen, Nalid und Nalmexon jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten-Komponenten, sofern eine Ausrüstung mit der Komponente (c) vorgesehen ist, in einer Menge von mindestens 1 mg, besonders bevorzugt in einer Menge von 3 bis 100 mg, ganz besonders bevorzugt in einer Menge von 5 bis 50 mg pro Darreichungsform, d.h. pro Dosiereinheit eingesetzt.

Vorzugsweise weist die erfindungsgemäße Darreichungsform die Antagonistenkomponente in einer üblichen, dem Fachmann bekannten therapeutischen Dosierung, besonders bevorzugt in einer dieser Dosierung gegenüber der üblichen Dosierung verdoppelten bis verdreifachten Menge pro Dosiereinheit auf.

Sofern die Kombination zur zusätzlichen Vorbeugung und Sicherung der erfindungsgemäßen Darreichungsform gegen Missbrauch die Komponente (d) umfaßt, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemäßer Anwendung keine Wirkung im Körper entfalten sollte.

Geeignete Emetika zur zusätzlichen Verhinderung des Missbrauchs der erfindungsgemäßen Darreichungsform sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

In die erfindungsgemäße Darreichungsform kommt bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z. B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäße Darreichungsform als Komponente (d) das Emetikum Emetin aufweisen, bevorzugt in einer Menge von wenigstens 3 mg, besonders bevorzugt wenigstens 10 mg und ganz besonders bevorzugt in einer Menge von wenigstens 20 mg pro Darreichungsform, d.h. Dosiereinheit.

Ebenfalls bevorzugt kann als Emetikum Apomorphin als zusätzliche Missbrauchssicherung zum Einsatz kommen, vorzugsweise in einer Menge von vorzugsweise wenigstens 3 mg, besonders bevorzugt wenigstens 5 mg und ganz besonders bevorzugt wenigstens 7 mg pro Dosiereinheit.

Sofern die erfindungsgemäße Darreichungsform die Komponente (e) als zusätzlichen missbrauchsverhindemden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffs eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäße Darreichungsform als zusätzlichen missbrauchsverhindemden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 zu entnehmen. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat, besonders bevorzugt wird Denatonium-Benzoat eingesetzt.

Zur Gewährleistung einer einmal täglichen Verabreichung weist die erfindungsgemäße Darreichungsform den Wirkstoff wenigstens zum Teil in retardierter Form auf, wobei die Retardierung der Wirkstofffreisetzung mit Hilfe von üblichen, dem Fachmann bekannten Materialien und Verfahren erzielt werden kann, beispielsweise durch Einbetten des Wirkstoffs in eine retardierende Matrix oder durch das Aufbringen eines oder mehrerer retardierender Überzüge. Die Wirkstoff-Abgabe muss aber so gesteuert sein, dass die vorstehend genannten Bedingungen jeweils erfüllt sind, z.B. das bei bestimmungsgemäßer Applikation der Darreichungsform das der Wirkstoff praktisch komplett freigesetzt wird, bevor die ggf. vorhandenen Komponente (c) und/oder (d) eine beeinträchtigende Wirkung entfalten können. Insbesondere muss die Freisetzung des Wirkstoffs eine analgetische Wirkung über mindestens 24 Stunden gewährleisten.

Sofern die Freisetzung des Wirkstoffs aus der erfindungsgemäßen Darreichungsform mit-Hilfe mindestens eines retardierenden Überzugs gesteuert wird, kann der retardierende Überzug aus üblichen, dem Fachmann bekannten Materialien bestehen.

In einer bevorzugten Ausführungsform der erfindungsgemäßen Darreichungsformen basiert der retardierende Überzug vorzugsweise auf einem wasserunlöslichen, gegebenenfalls modifizierten natürlichen und/oder synthetischen Polymeren oder auf einem natürlichen, halbsynthetischen oder synthetischen Wachs oder auf einem Fett oder einem Fettalkohol oder auf einem Gemisch aus wenigstens zwei der vorstehend genannten Komponenten.

Zur Herstellung eines retardierenden Überzugs werden als wasserunlösliche Polymere vorzugsweise Poly(meth)acrylate, besonders bevorzugt Poly(C₁₋₄)-alkyl(meth)acrylate, Poly(C₁₋₄)-dialkylamino-(C₁₋₄)-alkyl(meth)acrylate und/oder deren Copolymere, ganz besonders bevorzugt Copolymere aus Ethylacrylat und Methylmethacrylat mit einem molaren Verhältnis der Monomeren von 2 : 1 (Eudragit NE30D^{®}), Copolymere aus Ethylacrylat, Methylmethacrylat und Trimethylammoniumethylmethacrylat-chlorid mit einem molaren Verhältnis der Monomeren von 1 : 2 : 0,1 (Eudragit RS^{®}), Copolymere aus Ethylacrylat, Methylmethacrylat und Trimethylammoniumethylmethacrylatchlorid mit einem molaren Verhältnis der Monomeren von 1 : 2 : 0,2 (Eudragit RL^{®}) oder ein Gemisch aus wenigstens zwei dieser vorstehend genannten Copolymeren eingesetzt. Diese Überzugsmaterialien sind als 30 Gew.%-ige wäßrige Latexdispersionen, d.h. als Eudragit RS30D^{®}, Eudragit NE30D^{®} bzw. Eudragit RL30D^{®} am Markt erhältlich und werden als solche auch als Überzugsmaterial bevorzugt eingesetzt.

Ebenfalls bevorzugt können als wasserunlösliche Polymere zur Herstellung eines retardierenden Überzugs der erfindungsgemäßen Darreichungsformen Polyvinylacetate ggf. in Kombination mit weiteren Hilfstoffen eingesetzt werden. Diese sind als wäßrige Dispersion enthaltend 27 Gew.-% Polyvinylacetat, 2,5 Gew.-% Povidon und 0,3 Gew.-% Natriumlaurylsulfat (Kollicoat SR 30 D^{®}) am Markt erhältlich.

In einer weiteren bevorzugten Ausführungsform basieren die retardierenden Überzüge der erfindungsgemäßen Darreichungsform auf wasserunlöslichen Cellulosederivaten, vorzugsweise Alkylcellulosen wie z.B. Ethylcellulose, oder Celluloseestern, wie z.B. Celluloseacetat. Die Überzüge aus Ethylcellulose oder Celluloseacetat werden bevorzugt aus wäßriger Pseudolatexdispersion aufgebracht. Wäßrige Ethylcellulose-Pseudolatexdispersionen werden als 30 Gew.%-ige Dispersionen (Aquacoat^{®}) oder als 25 Gew.%-ige Dispersionen (Surelease^{®}) am Markt geführt.

Sofern der retardierende Überzug auf einem wasserunlöslichen, gegebenenfalls modifizierten natürlichen und/oder synthetischen Polymeren basiert, kann die Überzugsdispersion oder Lösung neben dem entsprechendem Polymer einen üblichen, dem Fachmann bekannten, physiologisch verträglichen Weichmacher aufweisen, um die notwendige Mindestfilmtemperatur zu senken.

Geeignete Weichmacher sind beispielsweise lipophile Diester aus einer aliphatischen oder aromatischen Dicarbonsäure mit C₆-C₄₀ und einem aliphatischen Alkohol mit C₁-C₈, wie z.B. Dibutylphthalat, Diethylphthalat, Dibutylsebacat oder Diethylsebacat, hydrophile oder lipophile Ester der Zitronensäure, wie z.B. Triethylcitrat, Tributylcitrat, Acetyltributylcitrat oder Acetyltriethylcitrat, Polyethylenglycole, Propylenglycol, Ester des Glycerins, wie z.B. Triacetin, Myvacet^{®} (acetylierte Mono- und Diglyceride, C₂₃H₄₄O₅ bis C₂₅H₄₇O₇), mittelkettige Triglyceride (Miglyol^{®}), Ölsäure oder Gemische aus wenigstens zwei der genannten Weichmacher. Vorzugsweise enthalten wäßrige Dispersionen von Eudragit RS^{®} und gegebenenfalls Eudragit RL^{®} Triethylcitrat.

Vorzugsweise enthält ein retardierender Überzug der erfindungsgemäßen Darreichungsform Weichmacher in Mengen von 5 bis 50 Gew-%., besonders bevorzugt 10 bis 40 Gew.-% und ganz besonders bevorzugt 10 bis 30 Gew-%, bezogen auf die Menge des eingesetzten Polymers. In Einzelfällen, beispielsweise für Celluloseacetat können auch höhere Mengen an Weichmachern eingesetzt werden.

Des weiteren kann ein retardierender Überzug weitere übliche, dem Fachmann bekannte Hilfsstoffe, wie z. B. Gleitmittel, vorzugsweise Talkum oder Glycerinmonostearat, Farbpigmente, vorzugsweise Eisenoxide oder Titandioxid, oder Tenside, wie z. B. Tween 80^{®} aufweisen.

Das Freisetzungsprofil des Wirkstoffs kann weiterhin durch übliche, dem Fachmann bekannte Möglichkeiten, wie z. B. durch die Dicke des Überzugs oder durch den Einsatz weiterer Hilfsstoffe als Bestandteile des Überzugs eingestellt werden. Geeignete Hilfsstoffe sind beispielsweise hydrophile oder pH-abhängige Porenbildner, wie z. B. Natrium-Carboxymethylcellulose, Celluloseacetatphthalat, Hydroxypropylmethylcelluloseacetatsuccinat, Lactose, Polyethylenglykol oder Mannitol oder wasserlösliche Polymere, wie z.B. Polyvinylpyrrolidon oder wasserlösliche Cellulosen, vorzugsweise Hydroxypropylmethylcellulose oder Hydroxypropylcellulose.

Die erfindungsgemäßen Darreichungsformen zur Freisetzung des zum Einsatz kommenden Wirkstoffs können zusätzlich auch einen magensaftresistenten Überzug aufweisen, der sich pH-abhängig auflöst. Durch diesen Überzug kann erreicht werden, dass die erfindungsgemäßen Darreichungsformen den Magentrakt unaufgelöst passieren und der Wirkstoffs erst im Darmtrakt zur Freisetzung gelangt.

Der magensaftresistente Überzug basiert vorzugsweise auf Methacrylsäure/Alkylmethacrylat-Copolymeren, vorzugsweise Methylmethacrylat wie Methacrylsäure oder Ethylmethacrylat-Copolymeren mit einem molaren Verhältnis der jeweiligen Monomeren von 1:1 bis 1:2, wie Eudragit L^{®}, Eudragit S^{®}, Eudragit L30D-55^{®}.

Ein Retardüberzug kann nach üblichen, dem Fachmann bekannten Verfahren, wie z. B. durch Aufsprühen von Lösungen, Dispersionen oder Suspensionen, durch Schmelzverfahren oder durch Pulverauftragsverfahren aufgebracht werden. Die Lösungen, Dispersionen oder Suspensionen können in Form von wäßrigen oder organischen Lösungen oder Dispersionen eingesetzt werden. Dabei werden wäßrige Dispersionen bevorzugt eingesetzt. Als organische Lösungsmittel können Alkohole, beispielsweise Ethanol oder Isopropanol, Ketone, wie z. B. Aceton, Ester, beispielsweise Ethylacetat, verwendet werden, wobei Alkohole und Ketone bevorzugt eingesetzt werden. Die Überzugsverfahren sind aus dem Stand der Technik, z. B. H. Sücker, Georg Thieme Verlag, 1991, Seiten 347 ff. bekannt.

Sofern die erfindungsgemäße Darreichungsformein multipartikulärer Form vorliegt, wird der retardierende Überzug vorzugsweise so aufgebracht, dass man die multipartikulären Formen enthaltend den Wirkstoff nach ihrer Herstellung mit den jeweiligen Polymeren und ggf. weiteren Hilfsstoffen aus wässrigen und/oder organischen Medien, vorzugsweise aus wässrigen Medien, mit Hilfe des Wirbelschichtverfahrens überzieht und den Überzug vorzugsweise gleichzeitig bei üblichen Temperaturen in der Wirbelschicht trocknet.

Vorzugsweise erfolgt die Trocknung eines Überzuges auf Poly(meth)acrylatbasis bei Temperaturen im Bereich von 30 bis 50 °C, besonders bevorzugt von 35 bis 45 °C. Für Überzüge auf Cellulosebasis, wie z. B. Ethylcellulose, erfolgt die Trocknung bevorzugt bei einer Temperatur im Bereich von 50 bis 80 °C, besonders bevorzugt im Bereich von 55 bis 65 °C. Wenn notwendig, kann sich an die Trocknung noch eine Temperung anschließen, um ein stabiles Freisetzungsprofil zu erhalten.

Die retardierte Freisetzung des Wirkstoffs aus der erfindungsgemäßen Darreichungsform kann auch durch Einbettung des Wirkstoffs in eine retardierende Matrix erzielt werden.

Als Materialien für eine retardierende Matrix können vorzugsweise physiologisch verträgliche, hydrophile Polymere, bevorzugt Celluloseether, Celluloseester und/oder Acrylharze verwendet werden. Besonders bevorzugt werden Ethylcellulose, Methylcellulose, Hydroxypropylmethylcellulose, Hydroxypropylcellulose, Hydroxyethylcellulose, Poly(meth)acrylsäure und/oder deren Derivate, wie deren Salze, Amide oder Ester eingesetzt.

Sofern hydrophobeverbindungen als Retardmatrix verwendet werden, können hydrophobe Polymere, Wachse, Fette, langkettige Fettsäuren, Fettalkohole oder entsprechende Ester oder Ether oder deren Gemische zum Einsatz kommen. Besonders bevorzugt werden als hydrophobe Verbindungen Mono- oder Diglyceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Matrix-Materialien einzusetzen.

Vorzugsweise kann die Komponente (b) als viskositätserhöhendes Mittel auch als Material für eine retardierende Matrix dienen, wenn dies der Aufbau der erfindungsgemäßen Darreichungsform zulässt.

Des weiteren kann auch die Komponente (C) und die ggf. vorhandene Komponente (D), die zur Erzielung der erfindungsgemäß notwendigen Bruchfestigkeit von mindestens 500 N, vorzugsweise mindestens 750 N, dienen, ggf. als zusätzliche retardierende Matrixmaterialien dienen.

Entsprechende retardierende Verbindungen und Verfahren zur Retardierung der erfindungsgemäßen Darreichungsformen sowie zum Aufbringen von geschmackskaschierenden und/oder magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

Die erfindungsgemäßen Darreichungsformen eignen sich zur oralen, vaginalen oder rektalen, vorzugsweise zur oralen, 1 x täglichen Einnahme bei Mensch und Tier.

Die erfindungsgemäße Darreichungsform kann in multipartikulärer Form, bevorzugt in Form von Mikrotabletten, Mikropellets, Granulaten, Sphäroiden, Perlen oder Pellets, ggf. in Kapseln abgefüllt oder zu Tabletten verpreßt, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform werden ggf. auch die üblichen Hilfsstoffe (B) zur Formulierung der Darreichungsform mitverwendet.

In einer weiteren bevorzugten Ausführungsform liegt die erfindungsgemäße Darreichungsform in Form einer Tablette, einer Kapsel oder in Form eines oralen osmotischen therapeutischen Systems (OROS) vor, vorzugsweise dann, wenn mindestens noch eine weitere missbrauchsverhindernde Komponente (a)-(f) vorhanden ist.

Die erfindungsgemäße, gegen Missbrauch gesicherte, feste Darreichungsform wird vorzugsweise hergestellt, indem die Komponenten (A), (C), ggf. (D), ggf. mindestens eine der zusätzlichen missbrauchsverhindemden Komponenten (a) - (f) und ggf. die weiteren Hilfsstoffe (B), wie vorzugsweise die retardierenden Matrix-Verbindungen gemischt werden, wobei die Komponenten (a)-(f), wenn notwendig, separat mit der Komponente (C) und ggf. (D) vermischt werden und die resultierende(n) Mischung(en) ggf. nach einer Granulierung zu der Darreichungsform unter vorangehender oder gleichzeitiger Wärmeeinwirkung durch Krafteinwirkung geformt wird (werden).

Die Granulierung kann gemäß einem Schmelzverfahren oder gemäß einer Feuchtgrarlulierung durchgeführt werden.

Diese Mischung(en) der Komponenten der erfindungsgemäßen Darreichungsform kann (können) in einem dem Fachmann bekannten Mischgerät erfolgen. Das Mischgerät kann beispielsweise ein Wälzmischer, Schüttelmischer, Schermischer oder Zwangsmischer sein.

Die resultierende(n) Mischung(en) wird vorzugsweise direkt durch Krafteinwirkung zu der erfindungsgemäßen Darreichungsform unter vorangehender und/oder gleichzeitiger Wärmeeinwirkung geformt. Beispielsweise kann die Mischung durch Direkttablettierung zu Tabletten geformt werden. Bei einer Direkttablettierung unter gleichzeitiger Wärmeeinwirkung wird das Tablettierwerkzeug, d. h. Unterstempel, Oberstempel und Matrize, zumindest bis zur Erweichung des Polymeren (C) kurz erhitzt und dabei verpreßt. Bei einer Direkttablettierung unter vorangehender Wärmeeinwirkung wird das zu verpreßende Gut unmittelbar vor der Tablettierung mindestens bis zur Erweichungstemperatur der Komponente (C) erhitzt und anschließend gepresst.

Die resultierende(n) Mischung(en) aus den Komponenten (A), (C), ggf. (D), den ggf. vorhandenen Komponenten (a) - (f) und ggf. weiteren Hilfsstoffe (B), insbesondere den retardierenden Matrix-Verbindungen, kann (können) auch zuerst granuliert, und anschließend unter vorangehender oder gleichzeitiger Wärmeeinwirkung zu der erfindungsgemäßen Darreichungsform unter Krafteinwirkung geformt werden.

Es ist auch möglich, die resultierende Mischung enthaltend den Wirkstoff und/oder eines oder mehrere seiner pharmazeutisch akzeptablen Salzen (A) sowie ggf. physiologisch verträgliche Hilfsstoffe (B) wie die Komponenten (a) bis (f) und ggf. der retardierenden Matrix-Verbindungen und mindestens ein synthetisches oder natürliches Polymer (C) und gegebenenfalls ein Wachs (D), unter einer Krafteinwirkung zu der Darreichungsform zu formen, gegebenenfalls die Formlinge zu vereinzeln und gegebenenfalls jeweils nach Größen zu separieren und nach oder während einer Erwärmung bis wenigstens zum Erweichungspunkt der Komponente (C) solange unter einer Krafteinwirkung zu belassen, bis die Formlinge eine Bruchhärte von mindestens 500 N, vorzugsweise mindestens 750 N, aufweisen, gegebenenfalls mit einer Umhüllung ggf. mit einem retardierenden Überzug zu versehen und die Formlinge gegebenenfalls alle wieder zu vermischen. Eine solche Verfahrensweise ist auch Gegenstand der internationalen Patentanmeldung PCT/EP2004/014679. Dem Fachmann ist geläufig, dass dabei durch mit Verwendung von Antioxidantien ggf. auf die Einhaltung einer Inertgasatmosphäre während des Herstellungsverfahrens verzichtet werden kann.

Darüber hinaus kann die notwendige Erwärmung der Mischung und/oder die Formlinge vor oder während der notwendigen Krafteinwirkung zur Erzielung der erfindungsgemäßen Bruchfestigkeit bzw. Härte von mindestens 500 N, vorzugsweise 750 N, mit Hilfe von Ultraschall erreicht werden. Eine entsprechende Verfahrensweise ist in der internationalen Patentanmeldung PCT/EP2005/004225 offenbart.

Sofern die Komponenten (c) und/oder (d) und/oder (f) in der erfindungsgemäßen Darreichungsform vorhanden sind, ist darauf zu achten, dass sie so formuliert oder so gering dosiert sind, dass sie bei bestimmungsgemäßer Applikation der Darreichungsform praktisch keine den Patienten oder die Wirksamkeit des Wirkstoffs beeinträchtigende Wirkung entfalten können.

Sofern die erfindungsgemäße Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung der Darreichungsform versehentlich, insbesondere durch Kinder, oder beim Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäßen Darreichungsform zur Sicherung der Darreichungsform enthaltend die Komponenten (c) und/oder (d) und/oder (f) vorgesehen ist, sollten diese Komponenten bevorzugt in einer so hohen Dosierung zum Einsatz kommen, dass sie bei einer mißbräuchlichen Applikation der Darreichungsform eine intensive negative Wirkung beim Missbraucher hervorrufen. Dies gelingt vorzugsweise durch eine räumliche Trennung zumindest des zum Einsatz kommenden Wirkstoffsvon den Komponenten (c) und/oder (d) und/oder (f), wobei bevorzugt der Wirkstoff in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Bei einer räumlichen Trennung in Untereinheit(en) (X) und Untereinheit(en) (Y) und unabhängig von der Anordnung dieser Untereinheiten in der Darreichungsform, enthält eine Untereinheit (X) den Wirkstoff in retardierter Form, so dass dieser in einer kontrollierten Freisetzung eine einmal tägliche Verabreichung gewährleistet.

Untereinheiten im Sinne der vorliegenden Erfindung sind feste Formulierungen, die jeweils neben üblichen, dem Fachmann bekannten Hilfsstoffen, den Wirkstoff, mindestens ein Polymer (C), ggf. ein Wachs (D) und gegebenenfalls wenigstens eine der gegebenenfalls vorhandenen Komponenten (a) und/oder (b) und/oder (e) bzw. jeweils wenigstens ein Polymer (C) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) sowie ggf. die retardierenden Matrix-Verbindungen enthalten. Dabei ist darauf zu achten, dass jede der Untereinheiten nach dem vorstehend angegebenen Verfahren formuliert werden.

Ein wesentlicher Vorteil der getrennten Formulierung des zum Einsatz kommenden Wirkstoffs von den Komponenten (c) bzw. (d) bzw. (f) in Untereinheiten (X) und (Y) der erfindungsgemäßen Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist.

Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C) und ggf. (D) enthalten und in der angegebenen Weise formuliert wurden.

Sollte es den Missbrauchem wider Erwarten gelingen, eine solche erfindungsgemäße Darreichungsform, welche die Komponenten (c) und/oder (e) und/oder (d) und/oder (f) in Untereinheiten (Y) aufweist, zum Zwecke der mißbräuchlichen Einnahme des Wirkstoffs zu zerkleinern und ein Pulver zu erhalten, das mit einem geeigneten Extraktionsmittel extrahiert werden soll, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung schon bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer erfindungsgemäßen Darreichungsform, in der eine räumliche Trennung des Wirkstoffs von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) einerseits und für die Freisetzung des Wirkstoffs, nämlich eine kontrollierte Freisetzung für eine einmal tägliche Verabreichung, andererseits, erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten (Y') in der erfindungsgemäßen Darreichungsform formuliert werden können, so lange die Sicherung der Darreichungsform gegen den Missbrauch wie auch die Wirkstoff-Freisetzung über 24 Stunden bei bestimmungsgemäßer Applikation durch die Art der Formulierung nicht beeinträchtig werden und das Polymer (C) mit formuliert und die Formulierung gemäß den vorstehend angegebenen Verfahren durchgeführt wird.

In einer bevorzugten Ausführungsform der erfindungsgemäßen Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y) durch mechanische Auslese möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multpartikulärer Form können auch bevorzugt in eine Kapsel abgefüllt oder zu einer Tablette verpreßt werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z. B. die Retardierung des Wirkstoffs oder eine magensaftresistente Ausrüstung und/oder Geschmackskaschierung der jeweiligen Untereinheiten.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäßen Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so dass neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäße Darreichungsform, in der die Untereinheit (Y) einen Kern bildet, der von der retardierten Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die der erfindungsgemäßen Härteanforderung genügen muss, in ein- und derselben multipartikulären Form formuliert sind.

In einer weiteren bevorzugten Ausführungsform der erfindungsgemäßen Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt:

Zwischen einer Schicht der Untereinheit (X) und einer Schicht der Untereinheit (Y) kann die erfindungsgemäße Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäße Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette, eines Coextrudats oder Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss die erfindungsgemäßen Härtevoraussetzungen erfüllen.

Ebenfalls besonders bevorzugt ist eine Ausführungsform der erfindungsgemäßen Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffs aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

Aus dem Stand der Technik ist dem Fachmann ebenfalls eine osmotische Darreichungsform enthaltend ein analgetisches Opioid und einen Farbstoff als aversives Mittel bekannt (WO 03/015531) bekannt. Vorzugsweise besteht der Tablettenkern aus zwei Schichten, einer opioidhaltigen Schicht und eine Push-Schicht, wobei die Push-Schicht den Farbstoff als aversives Mittel enthält.

In einer weiteren bevorzugten Ausführungsform hat die Untereinheit (X) der erfindungsgemäßen Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der Formulierung der erfindungsgemäßen Darreichungsform jeweils zum Einsatz kommenden Hilfsstoffe der Untereinheit(en) (X) bzw. (Y) sowie ggf. der vorhandenen Trennschicht(en) (Z) und/oder der Bartiereschicht(en) (Z') in Abhängigkeit von deren Anordnung in der erfindungsgemäßen Darreichungsform, der Applikationsart sowie in Abhängigkeit von dem vorhandenen Wirkstoff bzw. den ggf. vorhandenen Komponenten (a) und/oder (b) und/oder (e) und der Komponente (c) und/oder (d) und/oder (f) unter Einhaltung der Freisetzung des Wirkstoffs über 24 Stunden variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und ggf. die Komponente (D) zur Erfüllung der Härtebedingung der erfindungsgemäßen Darreichungsform enthält.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.

Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassen Alkylcellulosen, Hydroxyalkylcellulosen, Glucane, Skleroglucane, Mannane, Xanthane, Copolymere aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymere auf Basis von (Meth)acrylsäure sowie deren Estern, Polyamide, Polycarbonate, Polyalkylene, Polyalkylenglykole, Polyalkylenoxide, Polyalkylenterephtalate, Polyvinylalkohole, Polyvinylether, Polyvinylester, halogenierte Polyvinyle, Polyglykolide, Polysiloxane sowie Polyurethane, deren Copolymere oder Mischungen.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleirlsäuremonoethylester, Copolymere aus Methylvinylether und Maleinsäureanhydrid sowie Copolymere aus Vinylalkohol und Vinylacetat.

Weitere zur Formulierung der Barriereschicht geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäße Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, dass auch über die Dicke der Trennschicht die Freisetzung des Wirkstoffs bzw. der Komponente (c) und/oder (d) aus der jeweiligen Untereinheit mit gesteuert werden kann.

Die erfindungsgemäße Darreichungsform weist eine kontrollierte Freisetzung des Wirkstoffes über wenigstens 24 Stunden auf und eignet sich so zur 1x täglichen Verabreichung.

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Polymeren und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

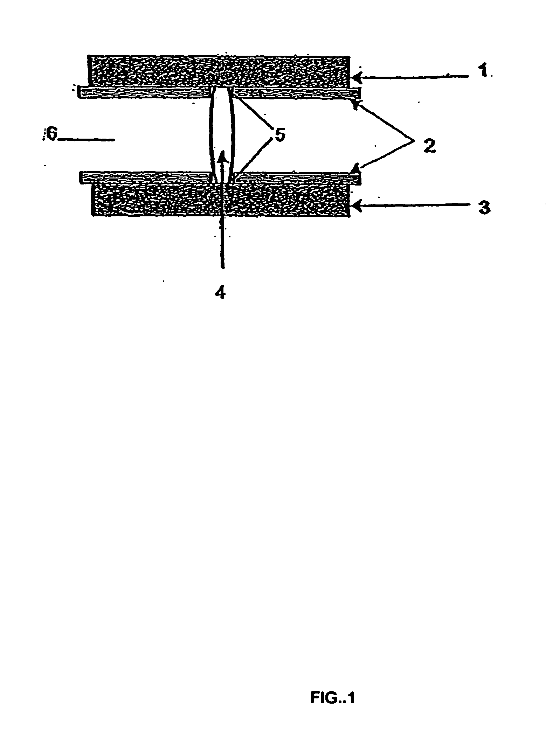

**Figur 1** zeigt die Messung der Bruchfestigkeit einer Tablette, insbesondere die dafür eingesetzte Justierungsvorrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar , aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiel 1

### Herstellung einer gegen Missbrauch gesicherten (1 R, 2R)-3-(3-dimethylamino-1-ethyl-2-methyl-propyl)-phenol-haltigen Tablette

Die in der Tabelle 1 aufgeführten Mengen an Wirkstoff-Hydrochlorid, Polyethylenoxidpulver und Hydroxypropylmethylcellulose (Metholose 90 SH 100 000) als Retardmatrixmaterial wurden in einem Freifallmischer gemischt. Das Tablettierwerkzeug, das aus Matrize, Ober- und Unterstempel mit einem Durchmesser von 13 mm bestand, wurde in einem Heizschrank auf 90°C erhitzt. Mittels des erhitzten Werkzeug wurden jeweils 600 mg der Pulvermischung verpresst, wobei der Pressdruck für mindestens 15 Sekunden aufrechterhalten wurde.

**Tabelle 1**

| Komponenten | Pro Tablette | Gesamtansatz |
|---|---|---|
| Wirkstoff-HCl | 200,0 mg | 60,0 g |
| Polyethylenoxid, NF, MG 7 000 000 (Polyox WSR 303, Dow Chemicals) | 360,0 mg | 138,0 g |
| Hydroxypropylmethylcellulose 100 000 mPas (Metholose 90 SH 100 000) | 40,0 mg | 12,0 g |
| Gesamtgewicht | 600,0 mg | 210,0 g |

Die Bruchfestigkeit der Tabletten wurde nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

### In-Vitro Freisetzung aus den hergestellten Tabletten

Die In-Vitro Freisetzung von Wirkstoff-Hydrochlorid aus den hergestellten Tabletten wurde in einer Blattrührerapparatur mit Sinker gemäß der in dem Europäischen Arzneibuch (European Pharmacopeia) beschriebenen Methode, bestimmt. Die Temperatur des Freisetzungsmediums betrug 37°C und die Umdrehungsgeschwindigkeit des Rühres 75 min⁻¹. Als Freisetzungsmedium wurden 600 ml Darmsaft pH 6,8 verwendet. Die jeweils zu einem Zeitpunkt im Lösungsmedium freigesetzte Menge an Wirkstoff-Hydrochlorid wurde spektralphotometrisch bestimmt Die prozentuale freigesetzte Menge, bezogen auf die Gesamtmenge Wirkostoff-Hydrochlorid, zu jedem Zeitpunkt ist in Tabelle 2 dargestellt.

**Tabelle 2**

| Zeit, min | Freigesetzte Menge, Gew.-% |
|---|---|
| 30 | 12 |
| 240 | 42 |
| 480 | 65 |
| 720 | 80 |
| 1080 | 94 |
| 1440 | 99 |

## Patentansprüche

1. Gegen Missbrauch gesicherte, orale Darreichungsform mit einer Bruchfestigkeit von mindestens 500 N und mit einer kontrollierten Freisetzung des Wirkstoffs (1R, 2R)-3-(3-dimethylamino-1-ethyl-2-methyl-propyl)-phenol für eine einmal tägliche Verabreichung, **dadurch gekennzeichnet, dass** sie den Wirkstoff und/oder mindestens eines seiner pharmazeutisch akzeptablen Salze oder denentsprechenden Ester, Ether und/oder das entsprechende Amid (A), mindestens ein synthetisches und/oder natürliches Polymer (C), ggf. wenigstens ein retardierendes MatrixMaterial und/oder ggf. wenigstens einen retardierenden Überzug, wenigstens einen weiteren physiologisch verträglichen Hilfsstoff (B), und gegebenenfalls wenigstens ein Wachs (D) umfasst.

2. Darreichungsform gemäß Anspruch 1, **dadurch gekennzeichnet, dass** als Salz das Hydrochlorid, Sulfat, Hydrobromid, Saccharinat, das Salz aus der Methansulfansäure, der Ameisensäure, der Essigsäure, der Oxalsäure, der Bernsteinsäure, Weinsäure, Mandelsäure, Fumarsäure, Milchsäure, Zitronensäure, Glutaminsäure oder Asparaginsäure und dem Wirkstoff vorliegt.

3. Darreichungsform gemäß Anspruch 1 oder 2, **dadurch gekennzeichnet, dass** als Salz das Hydrochlorid des Wirkstoffes vorliegt.

4. Darreichungsform gemäß Anspruch 1 bis 3, **dadurch gekennzeichnet, dass** die entsprechenden Stereoisomeren des Wirkstoffes vorliegen.

5. Darreichungsform gemäß einem der Ansprüche 1 bis 4, **dadurch gekennzeichnet, dass** sie in Form einer Tablette vorliegt.

6. Darreichungsform gemäß einem der Ansprüche 1 bis 4, **dadurch gekennzeichnet, dass** sie in multipartikulärer Form, vorzugsweise in Form von Mikrotabletten, Mikropellets, Granulaten, Sphäroiden, Perlen oder Pellets, ggf. zu Tabletten verpresst oder in Kapseln abgefüllt vorliegt.

7. Darreichungsform gemäß einem der Ansprüche 1 bis 6, **dadurch gekennzeichnet, dass** das Polymer (C) wenigstens ein Polymer ausgewählt aus der Gruppe umfassend Polyalkyenoxide, Polyethylene, Polypropylene. Polyvinylchloride, Polycarbonate, Polystyrole, Poly(meth)acrylate, deren Copolymerisate und Mischungen aus wenigstens zwei Vertretern der genannten Polymerklassen oder Polymeren ist.

8. Darreichungsform gemäß Anspruch 7, **dadurch gekennzeichnet, dass** das Polyalkylenoxid ein Polymethylenoxid, Polyethylenoxid und/oder Polypropylenoxid ist.

9. Darreichungsform nach einem der Ansprüche 1 bis 8, **dadurch gekennzeichnet, dass** als Polymer (C) ein hochmolekulares Polyethylenoxid vorliegt.

10. Darreichungsform gemäß einem der Ansprüche 1 bis 9, **dadurch gekennzeichnet, dass** das Polymer (C) ein wasserlösliches oder wasserquellbares Polymer ist.

11. Darreichungsform gemäß einem der Ansprüche 1 bis 10, **dadurch gekennzeichnet, dass** das Polyethylenoxid (C) ein Molekulargewicht von mindestens 0,5 Mio. aufweist.

12. Darreichungsform gemäß Anspruch 11, **dadurch gekennzeichnet, dass** das Molekulargewicht des Polyethylenoxids (C) mindestens 1 Mio. beträgt.

13. Darreichungsform gemäß Anspruch 11, **dadurch gekennzeichnet, dass** das Molekulargewicht des Polyethylenoxids (C) 1-15 Mio. beträgt.

14. Darreichungsform gemäß einem der Ansprüche 1 bis 12, **dadurch gekennzeichnet, dass** die Polymerkomponente (C) in einer Menge von wenigstens 20 Gew.%, vorzugsweise in einer Menge von 35 bis 99,9 Gew.%, besonders bevorzugt in einer Menge von wenigstens 50 Gew.%, ganz besonders bevorzugt von wenigstens 60 Gew.%, bezogen auf das Gesamtgewicht der Darreichungsform, zum Einsatz kommt.

15. Darreichungsform gemäß einem der Ansprüche 1 bis 14, **dadurch gekennzeichnet, dass** als Wachs (D) wenigstens ein natürliches, halbsynthetisches und/oder synthetisches Wachs mit einem Erweichungspunkt von wenigstens 60°C vorliegt.

16. Darreichungsform gemäß Anspruch 15, **dadurch gekennzeichnet, dass** das Wachs (D) Camaubawachs oder Bienenwachs ist.

17. Darreichungsform gemäß einem der Ansprüche 1 bis 16, **dadurch gekennzeichnet, dass** die Komponente(n) (C) und gegebenenfalls (D) in solchen Mengen vorliegt, dass die Darreichungsform eine Bruchfestigkeit von mindestens 500 N aufweist.

18. Darreichungsform gemäß einem der Ansprüche 1 bis 17, **dadurch gekennzeichnet, dass** der Wirkstoff in einer retardierenden Matrix vorliegt.

19. Darreichungsform gemäß Anspruch 18, **dadurch gekennzeichnet, dass** die Komponente (C) und/oder die Komponente (D) auch als retardierende Matrix-Komponente dient.

20. Darreichungsform gemäß einem der Ansprüche 1 bis 19, **dadurch gekennzeichnet, dass** wenigstens ein Hilfsstoff (B) als Material für die retardierende Matrix dient.

21. Darreichungsform gemäß einem der Ansprüche 1 bis 20, **dadurch gekennzeichnet, dass** sie einen Überzug, vorzugsweise eine reatardierenden und/oder geschmackskaschierenden Überzug, aufweist.

22. Darreichungsform gemäß einem der Ansprüche 1 bis 21, **dadurch gekennzeichnet, dass** sie als Hilfsstoff (B) wenigstens eine der nachfolgenden mißbrauchsverhindernden Komponenten (a)-(f) aufweist:
(a) wenigstens einen den Nasen- und/oder Rachenraum reizenden Stoff.
(b) wenigstens ein viskositätserhöhendes Mittel, das in einem mit Hilfe einer notwendigen Mindestmenge an einer wässrigen Flüssigkeit, vorzugsweise als ein aus der Darreichungsform gewonnenen Extrakt, ein Gel bildet, welches vorzugsweise beim Einbringen in eine weitere Menge einer wässrigen Flüssigkeit visuell unterscheidbar bleibt,
(c) wenigstens einen Antagonisten für den Wirkstoff mit Missbrauchspotential,
(d) wenigstens ein Emetikum,
(e) wenigstens einen Farbstoff als aversives Mittel,
(f) wenigstens einen Bitterstoff.

23. Darreichungsform gemäß Anspruch 22, **dadurch gekennzeichnet, dass** der Reizstoff gemäß Komponente (a) ein Brennen, einen Juckreiz, einen Niesreiz, eine vermehrte Sekretbildung oder eine Kombination mindestens zweier dieser Reize verursacht.

24. Darreichungsform gemäß Anspruch 22 oder 23, **dadurch gekennzeichnet, dass** der Reizstoff gemäß Komponente (a) auf einem oder mehreren Inhaltsstoffen wenigstens einer Scharfstoffdroge basiert.

25. Darreichungsform gemäß Anspruch 24, **dadurch gekennzeichnet, dass** die Scharfstoffdroge wenigstens eine Droge ausgewählt aus der Gruppe umfassend Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt wenigstens eine Droge ausgewählt aus der Gruppe umfassend Capsici Fructus (Paprika), Capsici Fructus acer (Cayennepfeffer) und Piperis nigri Fructus (Pfeffer) ist.

26. Darreichungsform gemäß Anspruch 24 oder 25, **dadurch gekennzeichnet, dass** der Inhaltsstoff der Scharfstoffdroge als eine o-Methoxy(Methyl)-phenol-Verbindung, eine Säureamid-Verbindung vorliegt, ein Senföl oder eine Sulfidverbindung ist oder sich von einer solchen Verbindung ableitet.

27. Darreichungsform gemäß einem der Ansprüche 24 bis 26, **dadurch gekennzeichnet, dass** der Inhaltsstoff der Scharfstoffdroge wenigstens ein Inhaltsstoff ausgewählt aus der Gruppe umfassend Myristicin, Elemicin, Isoeugenol, β-Asaron, Safrol, Gingerolen, Xanthorrhizol, Capsaicinoiden, vorzugsweise Capsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und eine von diesen Inhaltsstoffen abgeleiteten Verbindung ist.

28. Darreichungsform gemäß einem der Ansprüche 22 bis 27, **dadurch gekennzeichnet, dass** die Komponente (b) wenigstens ein viskositätserhöhendes Mittel ausgewählt aus der Gruppe umfassend mikrokristalline Cellulose mit 11 Gew.-% Carboxymethylcellulose-Natrium, Carboxymethylcellulose-Natrium, Polyacrylsäure, Johannisbrotkemmehl, Pektine aus Citrusfrüchten oder Äpfeln, Wachsmaisstärke, Natriumalginat, Guarkemmehl. Iota-Carrageen , Karaya Gummi, Gellangummi, Galaktomannan. Tarakernmehl, Propylenglykoalginat, Apfelpektin, Natrium-Hyaluronat, Tragant, Taragummi, fermentiertes Polysaccharid- Welan Gum und Xanthan-Gummi ist.

29. Darreichungsform gemäß einem der Ansprüche 22 bis 28, **dadurch gekennzeichnet**, das die Komponente (c) wenigstens ein Opioid-Antagonist ist.

30. Darreichungsform gemäß einem der Ansprüche 22 bis 29, **dadurch gekennzeichnet, dass** das Emetikum gemäß Komponente (d) auf einem oder mehreren Inhaltsstoffen von Radix Ipecacuanhae (Brechwurzel), vorzugsweise auf dem Inhaltsstoff Emetin basiert, und/oder Apomorphin ist.

31. Darreichungsform gemäß einem der Ansprüche 22 bis 30, **dadurch gekennzeichnet, dass** die Komponente (e) wenigstens ein physiologisch verträglicher Farbstoff ist.

32. Darreichungsform gemäß einem der Ansprüche 22 bis 31, **dadurch gekennzeichnet, dass** die Komponente (f) wenigstens ein Bitterstoff ausgewählt aus der Gruppe umfassend Aromaöle, vorzugsweise Pfefferminzöl, Eukalyptusöl, Bittermandelöl, Menthol und deren Mischungen, Fruchtaromastoffe, vorzugsweise von Zitronen, Orangen, Limonen, Grapefruit und deren Mischungen aus wenigstens 2 Komponenten, Denatoniumbenzoat und deren Mischungen aus wenigstens 2 Komponenten ist.

33. Darreichungsform gemäß einem der Ansprüche 22 bis 32, **dadurch gekennzeichnet, dass** der Wirkstoff (A) von der Komponente (c) und/oder (d) und/oder (f) räumlich getrennt, vorzugsweise ohne direkten Kontakt vorliegt, wobei der Wirkstoff bzw. die Wirkstoffe (A) bevorzugt in wenigstens einer Untereinheit (X) und die Komponenten (c) und/oder (d) und/oder (f) in wenigstens einer Untereinheit (Y) vorliegen und die Komponenten (c) und/oder (d) und/oder (f) aus der Untereinheit (Y) bei bestimmungsgemäßer Applikation der Darreichungsform im Körper bzw. bei Einnahme nicht ihre Wirkung entfalten.

34. Verfahren zur Herstellung einer Darreichungsform gemäß einem der Ansprüche 1 bis 33, **dadurch gekennzeichnet, dass** man
(1) die Komponenten (A), (C), ggf. (B) und gegebenenfalls (D) und ggf. retardierende Matrix-Verbindungen mischt, wobei man die ggf. vorhandenen Komponenten (a) bis (f), soweit notwendig, separat unter Zusatz der Komponente (C) und ggf. (D) mischt.
(2) die resultierende Mischung oder Mischungen ggf. nach einer Granulierung zu der Darreichungsform durch Krafteinwirkung und unter vorangehender oder gleichzeitiger Wärmeeinwirkung formt und ggf. mit einem retardierendem Überzug versieht.

35. Verfahren gemäß Anspruch 34, **dadurch gekennzeichnet, dass** die Granulierung gemäß einem Schmelzverfahren durchgeführt wird.

36. Verfahren gemäß Anspruch 34, **dadurch gekennzeichnet, dass** die Granulierung gemäß einer Feuchtgranulierung durchgeführt wird.

37. Verfahren zur Herstellung einer Darreichungsform gemäß einem der Ansprüche 1 bis 33, **dadurch gekennzeichnet, dass** man
(1) eine Mischung enthaltend die Komponenten (A), (C), ggf. (B) und ggf. (D) und ggf. retardierende Matrix-Verbindungen sowie die ggf. vorhandenen Komponenten (a) bis (f) ggf. als separate Mischung unter einer Krafteinwirkung zu Formlingen formt,
(2) gegebenenfalls die erhaltenen Formlinge vereinzelt und gegebenenfalls jeweils nach Größen separiert und
(3) nach oder während einer Erwärmung bis wenigstens zum Erweichung der Komponente (C) die Formlingen solange unter einer Krafteinwirkung lässt, bis die Formlinge eine Bruchhärte von mindestens 500 N aufweisen,
(4) gegebenenfalls mit einem Überzug, vorzugsweise einem retardierenden und/oder geschmackskaschierenden Überzug versieht und die Formlinge gegebenenfalls wieder vermischt.

38. Darreichungsform erhältlich nach Verfahren gemäß einem oder mehreren der Ansprüche 34 bis 37.

## Claims

1. Abuse-proofed, oral dosage form with a breaking strength of at least 500 N and with a controlled release of (1R,2R)-3-(3-dimethylamino-1-ethyl-2-methylpropyl)phenol for a once daily administration, **characterised in that** it comprises the active ingredient and/or at least one of its pharmaceutically acceptable salts or the corresponding ester, ether and/or the corresponding amide (A), at least one synthetic and/or natural polymer (C), optionally at least one delayed-release matrix material and/or optionally at least one delayed-release coating, at least one further physiologically acceptable auxiliary substance (B) and optionally at least one wax (D).

2. Dosage form according to claim 1, **characterised in that** the hydrochloride, sulphate, hydrobromide, saccharinate, the salt from the methanesulphonic acid, formic acid, acetic acid, oxalic acid, succinic acid, tartaric acid, mandelic acid, fumaric acid, lactic acid, citric acid, glutamic acid or aspartic acid and the active ingredient is present as salt.

3. Dosage form according to claim 1 or 2, **characterised in that** the hydrochloride of the active ingredient is present as salt.

4. Dosage form according to claims 1 to 3, **characterised in that** the corresponding stereoisomers of the active ingredient are present.

5. Dosage form according to one of claims 1 to 4, **characterised in that** it is in the form of a tablet.

6. Dosage form according to one of claims 1 to 4, **characterised in that** it is in multiparticulate form, preferably in the form of microtablets, micropellets, granules, spheroids, beads or pellets, optionally press-moulded into tablets or packaged in capsules.

7. Dosage form according to one of claims 1 to 6, **characterised in that** the polymer (C) is at least one polymer selected from among the group comprising polyalkylene oxides, polyethylenes, polypropylenes, polyvinyl chlorides, polycarbonates, polystyrenes, poly(meth)acrylates, the copolymers thereof and mixtures of at least two representatives of the stated polymer classes or polymers.

8. Dosage form according to claim 7, **characterised in that** the polyalkylene oxide is a polymethylene oxide, polyethylene oxide and/or polypropylene oxide.

9. Dosage form according to one of claims 1 to 8, **characterised in that** a polyethylene oxide of high molecular weight is present as polymer (C).

10. Dosage form according to one of claims 1 to 9, **characterised in that** the polymer (C) is a water-soluble or water-swellable polymer.

11. Dosage form according to one of claims 1 to 10, **characterised in that** the polyethylene oxide (C) has a molecular weight of at least 0.5 million.

12. Dosage form according to claim 11, **characterised in that** the molecular weight of the polyethylene oxide (C) is at least 1 million.

13. Dosage form according to claim 11, **characterised in that** the molecular weight of the polyethylene oxide (C) is 1-15 million.

14. Dosage form according to one of claims 1 to 12, **characterised in that** the polymer component (C) is used in a quantity of at least 20% by wt., preferably in a quantity of 35 to 99.9% by wt., particularly preferred in a quantity of at least 50% by wt., most particularly preferred of at least 60% by wt., relative to the total weight of the dosage form.

15. Dosage form according to one of claims 1 to 14, **characterised in that** the wax (D) is at least one natural, semi-synthetic and/or synthetic wax with a softening point of at least 60°C.

16. Dosage form according to claim 15, **characterised in that** the wax (D) is camauba wax or beeswax.

17. Dosage form according to one of claims 1 to 16, **characterised in that** the component(s) (C) and optionally (D) are present in such quantities that the dosage form exhibits a breaking strength of at least 500 N.

18. Dosage form according to one of claims 1 to 17, **characterised in that** the active ingredient is present in a delayed-release matrix.

19. Dosage form according to claim 18, **characterised in that** component (C) and/or component (D) also serves as the delayed-release matrix component.

20. Dosage form according to one of claims 1 to 19, **characterised in that** at least one auxiliary substance (B) serves as a material for the delayed-release matrix.

21. Dosage form according to one of claims 1 to 20, **characterised in that** it comprises a coating, preferably a delayed-release or taste-masking coating.

22. Dosage form according to one of claims 1 to 21, **characterised in that** it comprises at least one of the following abuse-preventing components (a)-(f) as auxiliary substance (B):
(a) at least one substance which irritates the nasal passages and/or pharynx,
(b) at least one viscosity-increasing agent, which, with the assistance of a necessary minimum quantity of an aqueous liquid, preferably as an aqueous extract obtained from the dosage form, forms a gel that preferably remains visually distinguishable when introduced into a further quantity of an aqueous liquid,
(c) at least one antagonist for the active ingredient with potential for abuse,
(d) at least one emetic,
(e) at least one dye as an aversive agent,
(f) at least one bitter substance.

23. Dosage form according to claim 22, **characterised in that** the irritant according to component (a) causes burning, itching, an urge to sneeze, increased formation of secretions or a combination of at least two of these stimuli.

24. Dosage form according to claim 22 or 23, **characterised in that** the irritant according to component (a) is based on one or more ingredients of at least one hot substance drug.

25. Dosage form according to claim 24, **characterised in that** the hot substance drug is at least one drug selected from the group consisting of Allii sativi bulbus, Asari rhizoma cum herba, Calami rhizoma, Capsici fructus (capsicum), Capsici fructus acer (cayenne pepper), Curcumae longae rhizoma, Curcumae xanthorrhizae rhizoma, Galangae rhizoma, Myristicae semen, Piperis nigri fructus (pepper), Sinapis albae semen (white mustard seed), Sinapis nigri semen, Zedoariae rhizoma and Zingiberis rhizoma, particularly preferred at least one drug from the group consisting of Capsici fructus (capsicum), Capsici fructus acer (cayenne pepper) and Piperis nigri fructus (pepper).

26. Dosage form according to claim 24 or 25, **characterised in that** the ingredient of the hot substance drug is present as an o-methoxy(methyl)phenol compound, an acid amide compound, a mustard oil or a sulphide compound or is derived from such a compound.

27. Dosage form according to one of claims 24 to 26, **characterised in that** the ingredient of the hot substance drug is at least one ingredient selected from the group consisting of myristicin, elemicin, isoeugenol, β-asarone, safrole, gingerols, xanthorrhizol, capsaicinoids, preferably capsaicin, piperine, preferably trans-piperine, glucosinolates, preferably based on non-volatile mustard oils, particularly preferred based on p-hydroxybenzyl mustard oil, methylmercapto mustard oil or methylsulphonyl mustard oil, and a compound derived from these ingredients.

28. Dosage form according to one of claims 22 to 27, **characterised in that** the component (b) is at least one viscosity-increasing agent selected from the group comprising microcrystalline cellulose with 11% by wt. carboxymethylcellulose sodium, carboxymethylcellulose sodium, polyacrylic acid, locust bean flour, pectins from citrus fruits or apples, waxy maize starch, sodium alginate, guar flour, iota-carrageenan, karaya gum, gellan gum, galactomannan, tara stone flour, propylene glycol alginate, apple pectin, sodium hyaluronate, tragacanth, tara gum, fermented polysaccharide welan gum and xanthan gum.

29. Dosage form according to one of claims 22 to 28, **characterised in that** the component (c) is at least one opioid antagonist.

30. Dosage form according to one of claims 22 to 29, **characterised in that** the emetic according to component (d) is based on one or more ingredients of ipecacuanha (ipecac) root, preferably on the ingredient emetine, and/or is apomorphine.

31. Dosage form according to one of claims 22 to 30, **characterised in that** the component (e) is at least one physiologically acceptable dye.

32. Dosage form according to one of claims 22 to 31, **characterised in that** the component (f) is at least one bitter substance selected from the group comprising aromatic oils, preferably peppermint oil, eucalyptus oil, bitter almond oil, menthol and mixtures thereof, fruit aroma substances, preferably from lemons, oranges, limes, grapefruit and mixtures thereof, and mixtures thereof of at least 2 components, denatonium benzoate and mixtures thereof of at least 2 components.

33. Dosage form according to one of claims 22 to 32, **characterised in that** the active ingredient (A) is present spatially separated from, preferably without direct contact with the component (c) and/or (d) and/or (f), wherein the active ingredient or the active ingredients (A) are preferably present in at least one subunit (X) and components (c) and/or (d) and/or (f) are present in at least one subunit (Y), and when the dosage form is correctly administered, components (c) and/or (d) and/or (f) from the subunit (Y) do not exert their effect in the body or on taking.

34. Process for the production of a dosage form according to one of claims 1 to 33, **characterised in that**
(1) components (A), (C), optionally (B) and optionally (D) and optionally delayed-release matrix compounds are mixed, wherein the optionally present components (a) to (f), if necessary, are mixed separately with addition of component (C) and optionally (D),
(2) the resultant mixture or mixtures, optionally after pelletisation, are formed into the dosage form by application of force and with preceding or simultaneous exposure to heat and are optionally provided with a delayed-release coating.

35. Process according to claim 34, **characterised in that** pelletisation is performed by a melt method.

36. Process according to claim 34, **characterised in that** pelletisation is performed by wet pelletisation.

37. Process for the production of a dosage form according to any one of claims 1 to 33, **characterised in that**
(1) a mixture containing components (A), (C), optionally (B) and optionally (D) and optionally delayed-release matrix compounds and the optionally present components (a) to (f) as a separate mixture is formed into shaped articles by application of force,
(2) the shaped articles obtained are optionally singulated and optionally graded by size in each case and
(3) after or during heating at least until component (C) softens, the shaped articles are exposed to action of force until the shaped articles exhibit a breaking hardness of at least 500 N,
(4) are optionally provided with a coating, preferably a delayed-release and/or taste-masking coating and the shaped articles are optionally all mixed together again.

38. Dosage form obtainable by processes according to one or more of claims 34 to 37.

## Revendications

1. Forme d'administration orale, assurée contre un usage abusif, présentant une résistance à l'écrasement d'au moins 500 N et une libération contrôlée de la substance active (1R,2R)-3-(3-diméthylamino-1-éthyl-2-méthylpropyl)-phénol destinée à une administration journalière unique, **caractérisée en ce qu'**elle comprend la substance active et/ou au moins un de ses sels pharmaceutiquement acceptables ou l'ester correspondant, l'éther correspondant et/ou l'amide correspondant (A), au moins un polymère synthétique et/ou naturel (C), le cas échéant au moins un matériau de matrice à effet de retard et/ou le cas échéant au moins un enrobage à effet de retard, au moins un autre adjuvant physiologiquement tolérable (B) et le cas échéant au moins une cire (D).

2. Forme d'administration selon la revendication 1, **caractérisée en ce que** le sel se trouve sous forme de chlorhydrate, de sulfate, de bromhydrate, de saccharinate, de sel de l'acide méthanesulfonique, de l'acide formique, de l'acide acétique, de l'acide oxalique, de l'acide succinique, de l'acide tartrique, de l'acide mandélique, de l'acide fumarique, de l'acide lactique, de l'acide citrique, de l'acide glutamique ou de l'acide aspartique de la substance active.

3. Forme d'administration selon la revendication 1 ou 2, **caractérisée en ce que** le sel se trouve sous forme de chlorhydrate de la substance active.

4. Forme d'administration selon la revendication 1 à 3, **caractérisée en ce que** les stéréo-isomères correspondants de la substance active sont présents.

5. Forme d'administration selon l'une quelconque des revendications 1 à 4, **caractérisée en ce qu'**elle se trouve sous forme d'un comprimé.

6. Forme d'administration selon l'une quelconque des revendications 1 à 4, **caractérisée en ce qu'**elle se trouve sous forme multiparticulaire, de préférence sous forme de microcomprimés, de micropellets, de granulats, de sphéroïdes, de billes ou de pellets, le cas échéant pressée en comprimés ou transvasée dans des capsules.

7. Forme d'administration selon l'une quelconque des revendications 1 à 6, **caractérisée en ce que** le polymère (C) est au moins un polymère choisi dans le groupe comprenant les poly(oxydes d'alkylène), les polyéthylènes, les polypropylènes, les poly(chlorures de vinyle), les polycarbonates, les polystyrènes, les poly(méth)acrylates, leurs copolymères et mélanges d'au moins deux représentants des classes de polymères ou des polymères mentionné(e)s.

8. Forme d'administration selon la revendication 7, **caractérisée en ce que** le poly(oxyde d'alkylène) est un poly(oxyde de méthylène), un poly(oxyde d'éthylène) et/ou un poly(oxyde de propylène).

9. Forme d'administration selon l'une quelconque des revendications 1 à 8, **caractérisée en ce que** le polymère (C) se trouve sous forme d'un poly(oxyde d'éthylène) de haut poids moléculaire.

10. Forme d'administration selon l'une quelconque des revendications 1 à 9, **caractérisée en ce que** le polymère (C) est un polymère soluble ou gonflable dans l'eau.

11. Forme d'administration selon l'une quelconque des revendications 1 à 10, **caractérisée en ce que** le poly(oxyde d'éthylène) (C) présente un poids moléculaire d'au moins 0,5 million.

12. Forme d'administration selon la revendication 11, **caractérisée en ce que** le poids moléculaire du poly(oxyde d'éthylène) (C) est d'au moins 1 million.

13. Forme d'administration selon la revendication 11, **caractérisée en ce que** le poids moléculaire du poly(oxyde d'éthylène) (C) est de 1-15 millions.

14. Forme d'administration selon l'une quelconque des revendications 1 à 12, **caractérisée en ce que** le composant polymère (C) est utilisé en une quantité d'au moins 20% en poids, de préférence en une quantité de 35 à 99,9% en poids, de manière particulièrement préférée d'au moins 50% en poids, de manière tout particulièrement préférée d'au moins 60% en poids, par rapport au poids total de la forme d'administration.

15. Forme d'administration selon l'une quelconque des revendications 1 à 14, **caractérisée en ce que** la cire (D) se trouve sous forme d'au moins une cire naturelle, semi-synthétique et/ou synthétique présentant un point de ramollissement d'au moins 60°C.

16. Forme d'administration selon la revendication 15, **caractérisée en ce que** la cire (D) est la cire de caroube ou la cire d'abeille.

17. Forme d'administration selon l'une quelconque des revendications 1 à 16, **caractérisée en ce que** le(s) composant (s) (C) et le cas échéant (D) se trouve (nt) en des quantités telles que la forme d'administration présente une résistance à l'écrasement d'au moins 500 N.

18. Forme d'administration selon l'une quelconque des revendications 1 à 17, **caractérisée en ce que** la substance active se trouve dans une matrice à effet de retard.

19. Forme d'administration selon la revendication 18, **caractérisée en ce que** le composant (C) et/ou le composant (D) ser(ven)t également de composant de matrice à effet de retard.

20. Forme d'administration selon l'une quelconque des revendications 1 à 19, **caractérisée en ce qu'**au moins un adjuvant (B) sert de matériau pour la matrice à effet de retard.

21. Forme d'administration selon l'une quelconque des revendications 1 à 20, **caractérisée en ce qu'**elle présente un enrobage, de préférence un enrobage à effet de retard et/ou de masquage du goût.

22. Forme d'administration selon l'une quelconque des revendications 1 à 21, **caractérisée en ce qu'**elle présente, comme adjuvant (B), au moins un des composants suivants, empêchant l'usage abusif (a)-(f) :
(a) au moins une substance irritant le nez et/ou la gorge,
(b) au moins un agent qui augmente la viscosité, qui forme dans un extrait de préférence obtenu à partir de la forme d'administration, à l'aide d'une quantité minimale nécessaire d'un liquide aqueux, un gel, qui reste de préférence visuellement détectable lors de l'introduction d'une autre quantité de liquide aqueux,
(c) au moins un antagoniste de la substance active présentant un potentiel d'usage abusif,
(d) au moins un émétique
(e) au moins un colorant comme agent d'aversion
(f) au moins une substance amère.

23. Forme d'administration selon la revendication 22, **caractérisée en ce que** la substance irritante selon le composant (a) provoque une brûlure, une démangeaison, une irritation sternutatoire, une sécrétion accrue ou une combinaison d'au moins deux de ces irritations.

24. Forme d'administration selon la revendication 22 ou 23, **caractérisée en ce que** la substance irritante selon le composant (a) est à base d'un ou de plusieurs constituants d'au moins un extrait piquant.

25. Forme d'administration selon la revendication 24, **caractérisée en ce que** l'extrait piquant est au moins un extrait choisi dans le groupe comprenant Allii sativi Bulbus, Asari Rhizoma c. Herba, Calami Rhizoma, Capsici Fru,ctus (paprika), Capsici Fructus acer (poivre de Cayenne), Curcumae longae Rhizoma, Curcumae xanthorrhizae Rhizoma, Galangae Rhizoma, Myristicae Semen, Piperis nigri Fructus (poivre), Sinapis albae (Erucae) Semen, Sinapis nigri Semen, Zedoariae Rhizoma et Zingiberis Rhizoma, de manière particulièrement préférée un extrait choisi dans le groupe comprenant Capsici Fructus (paprika), Capsici Fructus acer (poivre de Cayenne) et Piperis nigri Fructus (poivre).

26. Forme d'administration selon la revendication 24 ou 25, **caractérisée en ce que** le constituant de l'extrait piquant se trouve sous forme d'un composé o-méthoxy(méthyl)-phénol, un composé amide d'acide, une huile de moutarde ou un composé de type sulfure ou est dérivé d'un tel composé.

27. Forme d'administration selon l'une quelconque des revendications 24 à 26, **caractérisée en ce que** le constituant de l'extrait piquant est au moins un constituant choisi dans le groupe comprenant la myristicine, l'élémicine, l'isoeugénol, la ß-asarone, le safrol, les gingérols, le xanthorrhizol, les capsaïcinoïdes, de préférence la capsaïcine, la pipérine, de préférence la trans-pipérine, les glucosinolates, de préférence à base d'huiles de moutarde non volatiles, de manière particulièrement préférée à base de p-hydroxybenzyl-huile de moutarde, de méthylmercapto-huile de moutarde ou de méthylsulfonyl-huile de moutarde, et un composé dérivé de ces constituants.

28. Forme d'administration selon l'une quelconque des revendications 22 à 27, **caractérisée en ce que** le composant (b) est au moins un agent augmentant la viscosité, choisi dans le groupe comprenant la cellulose microcristalline à 11 % en poids de carboxyméthylcellulose sodique, la carboxyméthylcellulose sodique, un poly(acide acrylique), la farine de graines de caroube, les pectines d'agrumes ou de pommes, l'amidon de maïs cireux, l'alginate de sodium, la farine de graines de guar, l'iota-carraghénane, la gomme karaya, la gomme gellane, le galactomannane, la farine de graines de tara, le propylèneglyco-alginate, la pectine de pomme, l'hyaluronate sodique, la gomme adragante, la gomme tara, la gomme welan-polysaccharide fermenté et la gomme xanthane.

29. Forme d'administration selon l'une quelconque des revendications 22 à 28, **caractérisée en ce que** le composant (c) est au moins un antagoniste d'opioïde.

30. Forme d'administration selon l'une quelconque des revendications 22 à 29, **caractérisée en ce que** l'émétique selon le composant (d) est à base d'un ou de plusieurs constituants du Radix Ipecacuanhae (racine d'ipéca), de préférence du constituant émétine et/ou est l'apomorphine.

31. Forme d'administration selon l'une quelconque des revendications 22 à 30, **caractérisée en ce que** le composant (e) est au moins un colorant physiologiquement acceptable.

32. Forme d'administration selon l'une quelconque des revendications 22 à 31, **caractérisée en ce que** le composant (f) est au moins une substance amère choisie dans le groupe comprenant les huiles essentielles, de préférence l'huile essentielle de menthe poivrée, l'huile essentielle d'eucalyptus, l'huile essentielle d'amande amère, le menthol et leurs mélanges, les arômes de fruits, de préférence de citrons, d'oranges, de citrons verts, de pamplemousse et leurs mélanges d'au moins deux composants, le benzoate de dénatonium et leurs mélanges d'au moins deux composants.

33. Forme d'administration selon l'une quelconque des revendications 22 à 32, **caractérisée en ce que** la substance active (A) est présente séparée dans l'espace du composant (c) et/ou (d) et/ou (f), de préférence sans contact direct, la substance active ou les substances actives (A) se trouvant de préférence dans au moins une sous-unité (X) et les composants (c) et/ou (d) et/ou (f) se trouvant dans au moins une sous-unité (Y) et les composants (c) et/ou (d) et/ou (f) ne déployant pas leur action à partir de la sous-unité (Y) lors de l'administration ou lors de la prise prescrite de la forme d'administration dans le corps.

34. Procédé pour la préparation d'une forme d'administration selon l'une quelconque des revendications 1 à 33, **caractérisé en ce qu'**on
(1) mélange les composants (A), (C), le cas échéant (B) et le cas échéant (D) et le cas échéant les composés de matrice à effet retard, en mélangeant les composants (a) à (f) le cas échéant présents, si nécessaire, séparément avec addition du composant (C) et le cas échéant du composant (D),
(2) moule le mélange résultant ou les mélanges résultants, le cas échéant après une granulation, en la forme d'administration sous l'effet d'une force et sous l'effet, préalable ou simultané, de la chaleur et la munit le cas échéant d'un enrobage à effet retard.

35. Procédé selon la revendication 34, **caractérisé en ce que** la granulation est réalisée selon un procédé en masse fondue.

36. Procédé selon la revendication 34, **caractérisé en ce que** la granulation est réalisée selon une granulation humide.

37. Procédé pour la préparation d'une forme d'administration selon l'une quelconque des revendications 1 à 33, **caractérisé en ce qu'**on
(1) moule en pièces façonnées un mélange contenant les composants (A), (C), le cas échéant (B) et le cas échéant (D) et le cas échéant les composés de matrice à effet retard ainsi que les composants (a) à (f) le cas échéant présents, le cas échéant en tant que mélange séparé, sous l'action d'une force,
(2) individualise le cas échéant les pièces façonnées obtenues et les sépare le cas échéant en fonction des tailles et
(3) laisse les pièces façonnées sous l'effet d'une force après ou pendant un chauffage au moins jusqu'au ramollissement du composant (C), jusqu'à ce que les pièces façonnées présentent une résistance à l'écrasement d'au moins 500 N,
(4) les munit le cas échéant d'un enrobage, de préférence d'un enrobage à effet retard et/ou masquant le goût et mélange le cas échéant à nouveau les pièces façonnées.

38. Forme d'administration pouvant être obtenue selon un procédé selon l'une ou plusieurs des revendications 34 à 37.
